(19) Europäisches Patentamt
European Patent Office
Office européen des brevets

(11) Veröffentlichungsnummer : **0 475 908 A1**

(12) **EUROPÄISCHE PATENTANMELDUNG**

(21) Anmeldenummer : **91810713.7**

(22) Anmeldetag : **05.09.91**

(51) Int. Cl.$^5$ : **C07D 487/04,** C07D 498/04, C07D 513/04, B41M 5/26, B41M 5/28, B41M 5/34

(30) Priorität : **14.09.90 CH 2989/90**
**13.06.91 CH 1759/91**

(43) Veröffentlichungstag der Anmeldung : **18.03.92 Patentblatt 92/12**

(84) Benannte Vertragsstaaten : **AT BE CH DE ES FR GB IT LI**

(71) Anmelder : **CIBA-GEIGY AG**
**Klybeckstrasse 141**
**CH-4002 Basel (CH)**

(72) Erfinder : **Baumann, Hans, Dr.**
**Rohrhagstrasse 16**
**CH-4104 Oberwil (CH)**
Erfinder : **Fletcher, Ian John, Dr.**
**Bürgenstal 27**
**CH-4312 Magden (CH)**

(54) **Chromogene Lactamverbindungen und ihre Herstellung und Verwendung.**

(57) Chromogene Lactamverbindungen der Formel

(1)

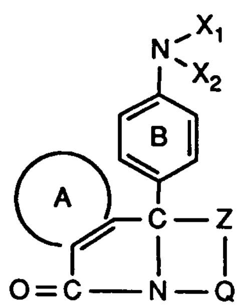

worin

der Ring A einen aromatischen oder heteroaromatischen Rest mit 6 Ringatomen, der einen aromatischen annellierten Ring aufweisen kann, wobei sowohl der Ring A als auch der annellierte Ring substituiert sein können ;

der Ring B einen unsubstituierten oder substituierten Benzolkern ;

Z -NR, O oder S;

R Wasserstoff ; unsubstituiertes oder substituiertes $C_1$-$C_{12}$-Alkyl ; Cycloalkyl mit 5 bis 10 Kohlenstoffatomen ; unsubstituiertes oder substituiertes Aryl oder Aralkyl ; $C_1$-$C_{12}$-Acyl ; N-Niederalcarbamoyl ; oder gegebenenfalls ringsubstituiertes N-Arylcarbamoyl ;

Q $C_1$-$C_{12}$-Alkylen, Aryl-$C_1$-$C_4$-Alkylen, 1,2-Cycloalkylen, 1,2- oder 1,8-Arylen oder Aralkylen ; und

$X_1$ und $X_2$, unabhängig voneinander, je Wasserstoff ; unsubstituiertes oder substituiertes Alkyl mit höchstens 12 Kohlenstoffatomen ; Acyl mit 1 bis 8 Kohlenstoffatomen ; Cycloalkyl mit 5 bis 10 Kohlenstoffatomen ; oder unsubstituiertes oder ringsubstituiertes Aralkyl oder Aryl ; oder ($X_1$ und $X_2$) zusammen mit dem gemeinsamen Stickstoffatom einen fünf- oder sechsgliedrigen, vorzugsweise gesättigten, heterocyclischen Rest
bedeutet.

Diese Lactamverbindungen eignen sich insbesondere als Farbbildner in druck- oder wärmeempfindlichen Aufzeichnungsmaterialien und ergeben lichtechte gelbe, rote, violette, blaue, grünblaue oder grüne Farbtöne.

EP 0 475 908 A1

Die vorliegende Erfindung betrifft chromogene Lactamverbindungen, Verfahren zu ihrer Herstellung und ihre Verwendung in druck- oder wärmeempfindlichen Aufzeichnungsmaterialien.

Die erfindungsgemässen Lactamverbindungen entsprechen der allgemeinen Formel

(1) 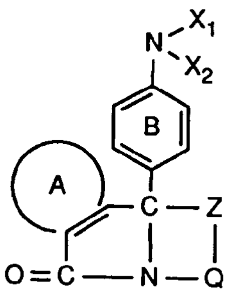

worin

der Ring A einen aromatischen oder heteroaromatischen Rest mit 6 Ringatomen, der einen aromatischen annellierten Ring aufweisen kann, wobei sowohl der Ring A als auch der annellierte Ring substituiert sein können;

der Ring B einen unsubstituierten oder durch Halogen, Niederalkyl, $C_1$-$C_{12}$-Alkoxy, besonders Niederalkoxy, Benzyloxy, Niederalkylcarbonylamino, Mono- oder Diniederalkylamino substituierten Benzolkern;

Z -NR, O oder S;

R Wasserstoff; unsubstituiertes oder durch Halogen, Hydroxy, Cyano, Benzoyl, $C_1$-$C_{12}$-Acyl, Diniederalkylamino oder Niederalkoxy substituiertes $C_1$-$C_{12}$-Alkyl; Cycloalkyl mit 5 bis 10 Kohlenstoffatomen; unsubstituiertes oder durch Halogen, Cyano, Nitro, $C_1$-$C_4$-Halogenalkyl, Niederalkyl, Niederalkoxy, Niederalkoxycarbonyl oder $C_1$-$C_{12}$-Acyl ringsubstituiertes Aryl oder Aralkyl; $C_1$-$C_{12}$-Acyl; N-Niederalkcarbamoyl; oder gegebenenfalls ringsubstituiertes N-Arylcarbamoyl;

Q $C_1$-$C_{12}$-Alkylen, Aryl-$C_1$-$C_4$-Alkylen, 1,2-Cycloalkylen, 1,2- oder 1,8-Arylen oder Aralkylen; und

$X_1$ und $X_2$, unabhängig voneinander, je Wasserstoff; unsubstituiertes oder durch Halogen, Hydroxy, Cyano, Tetrahydrofuryl oder Niederalkoxy substituiertes Alkyl mit höchstens 12 Kohlenstoffatomen; Acyl mit 1 bis 8 Kohlenstoffatomen; Cycloalkyl mit 5 bis 10 Kohlenstoffatomen; oder unsubstituiertes oder durch Halogen, Cyano, Nitro, Trifluormethyl, Niederalkyl, Niederalkoxy, Niederalkoxycarbonyl, -NX′X″ oder 4-X′X″N-phenylamino ringsubstituiertes Aralkyl oder Aryl, worin X′ und X″, unabhängig voneinander, Wasserstoff, Niederalkyl, Cyclohexyl, Benzyl oder Phenyl darstellen; oder ($X_1$ und $X_2$) zusammen mit dem gemeinsamen Stickstoffatom einen fünf- oder sechsgliedrigen, vorzugsweise gesättigten, heterocyclischen Rest bedeuten.

Als 6-gliedriger aromatischer Ring stellt A vorzugsweise einen Benzolring dar, der unsubstituiert oder durch Halogen, Cyano, Nitro, Niederalkyl, Niederalkoxy, Niederalkylthio, Niederalkycarbonyl, Niederalkoxycarbonyl, Amino, Niederalkylamino, Diniederalkylamino oder Niederalkylcarbonylamino substituiert ist. Als 6-gliedriger heterocyclischer Ring stellt A insbesondere einen stickstoffhaltigen Heterocyclus mit aromatischem Charakter, wie z.B. einen Pyridin- oder Pyrazinring dar. Der Ring A kann auch einen annellierten aromatischen Ring, vorzugsweise einen Benzolring enthalten und stellt somit z.B. einen Naphthalin-, Chinolin- oder Chinoxalinring dar.

Die durch A wiedergegebenen bevorzugten 6-gliedrigen aromatischen oder heterocyclischen Reste sind der 2,3-Pyridino-, 3,4-Pyridino-, 2,3-Pyrazino-, 2,3-Chinoxalino-, 1,2-Naphthalino-, 2,3-Naphthalino- oder 1,2-Benzorest, der unsubstituiert oder durch Halogen, wie Chlor oder Brom, Nitro, Niederalkyl, Niederalkoxy, Niederalkylthio oder eine wie vorstehend definiert gegebenenfalls substituierte Aminogruppe substituiert ist, wobei der unsubstituierte oder durch Chloratome, Niederalkoxy oder vorzugsweise Diniederalkylamino, besonders durch Dimethylamino oder Diethylamino substituierte 1,2-Benzorest besonders bevorzugt ist.

Der Ring B stellt vorteilhafterweise einen unsubstituierten oder durch Halogen, Niederalkyl, Niederalkoxy, Acetylamino oder Diniederalkylamino substituierten Phenylenrest dar. Besonders bevorzugt ist der Ring B ein unsubstituierter oder durch Niederalkoxy substituierter Phenylenrest.

Vorteilhafterweise bedeutet Z-S-, -O- oder

-NR',

worin R′ Wasserstoff, Niederalkyl, Cyano-Niederalkyl, Niederalkylcarbonyl wie Acetyl, Phenyl, Benzyl, N-Niederalkylcarbamoyl oder gegebenenfalls durch Halogen, Nitro, Trifluormethyl, Niederalkyl, Niederalkoxy oder

Niederalkoxycarbonyl substituiertes N-Phenylcarbamoyl (Carbanilino) darstellt. Vorzugsweise ist

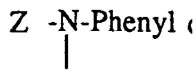

oder vor allem -NH-.

Q in der Bedeutung eines Alkylenrestes weist vorteilhafterweise 2 bis 6 Kohlenstoffatome vorzugsweise 2 oder 3 Kohlenstoffatome auf und kann geradkettig oder verzweigt sein. Es handelt sich beispielsweise um die $-CH_2CH_2-$,

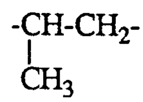 , 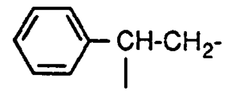 ,

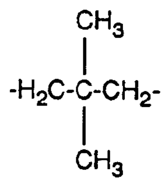 Gruppe

und besonders um die $-CH_2CH_2CH_2-$-Gruppe. Q als verzweigter Alkylenrest umfasst ausserdem spirocyclisch anellierte Reste, wie den

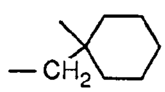 ,

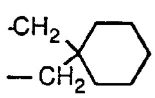 , 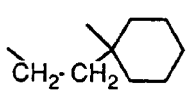 , 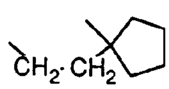 , 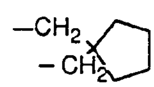

oder

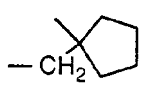

Rest, die unsubstituiert oder ihrerseits bis zu dreifach durch $C_1$-$C_4$-Alkyl substituiert sein können.

Als Cycloalkylen für Q kommt vor allem die 1,2-Cyclohexylengruppe und 1,2-Cyclopentylen, bevorzugt 1,2-Cyclohexylen, in Frage.

Als Arylen stellt Q vorzugsweise einen 1,2-Phenylenrest dar, der unsubstituiert oder durch Halogen, Methyl oder Methoxy substituiert ist. Vorteilhafterweise kann Q als Arylen ein 1,8-Naphthylenrest oder ein Diphenylenrest darstellen.

Vorzugsweise bedeutet Q 1,2-Phenylen, 1,2-Cyclohexylen, 1,8-Naphthylen, 1,2-Ethylen ($-CH_2CH_2-$), Neopentylen

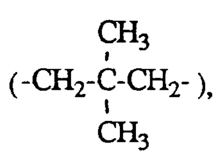

1,2-Propylen

$$\begin{array}{l}(-\underset{|}{C}HCH_2-)\\ \;\;CH_3\end{array}$$

oder vor allem 1,3-Propylen $(-CH_2-CH_2-CH_2-)$.

Stellen die Substituenten R, $X_1$ und $X_2$ Alkylgruppen dar, so können sie geradkettig oder verzweigt sein. Beispiele solcher Alkylreste sind Methyl, Ethyl, n-Propyl, Isopropyl, n-Butyl, 1-Methylbutyl, sek.-Butyl, tert.-Butyl, Amyl, Isoamyl, n-Hexyl, 2-Ethyl-hexyl, n-Heptyl, n-Octyl, Isooctyl, 1,1,3,3-Tetramethylbutyl, n-Nonyl, Isononyl, 3-Ethylheptyl, Decyl oder n-Dodecyl.

Sind die Alkylreste in R, $X_1$ und $X_2$ substituiert, so handelt es sich vor allem um Cyanoalkyll, Halogenalkyl, Hydroxyalkyl, Niederalkoxyalkyl jeweils vorzugsweise mit insgesamt 2 bis 8 Kohlenstoffatomen, wie z.B. 2-Cyanoethyl, 2-Chlorethyl, 2-Chlorpropyl, 2-Hydroxyethyl, 3-Hydroxypropyl, 2-Methoxyethyl, 2-Ethoxyethyl, 2,3-Dihydroxypropyl, 2-Hydroxy-2-chlorpropyl, 3-Methoxypropyl, 4-Methoxybutyl, Trichlormethyl, Trifluormethyl, Tetrafluoroethyl, Tetrachlorethyl oder 4-Propoxybutyl sowie bei $X_1$ und $X_2$ auch Tetrahydrofurfuryl.

Beispiele für Cycloalkyl in der Bedeutung von R, $X_1$ und $X_2$ sind Cyclopentyl, Cycloheptyl oder vorzugsweise Cyclohexyl. Die Cycloalkylreste können einen oder mehrere $C_1$-$C_4$-Alkylreste, vorzugsweise Methylgruppen, enthalten und weisen insgesamt 5 bis 10 Kohlenstoffatome auf.

Als Aralkyl können R, $X_1$ und $X_2$ Phenethyl, Phenylisopropyl oder vor allem Benzyl sein. Als Aryl stellen die X-Reste, sowie auch der Substituent R besonders Naphthyl oder in erster Linie Phenyl dar.

Bevorzugte Substituenten in der Aralkyl- und Arylgruppe der X-Reste sind z.B. Halogen, Cyano, Methyl, Trifluormethyl, Methoxy oder Carbomethoxy. Bevorzugte Substituenten im Arylrest R sind z.B. Halogen, Methyl oder Methoxy. Beispiele für derartige araliphatische bzw. aromatische Reste sind Methylbenzyl, 2,4- oder 2,5-Dimethylbenzyl, Chlorbenzyl, Dichlorbenzyl, Cyanobenzyl, Tolyl, Xylyl, Chlorophenyl, Methoxyphenyl, Trifluormethylphenyl, 2,6-Dimethylphenyl oder Carbomethoxyphenyl.

Wenn $X_1$ und $X_2$ zusammen mit dem gemeinsamen Stickstoffatom einen heterocyclischen Rest darstellen, so ist dieser beispielsweise Pyrrolidino, Piperidino, Pipecolino, Morpholino, Thiomorpholino oder Piperazino; z.B. N-Methylpiperazino oder N-Phenylpiperazino. Bevorzugte gesättigte heterocyclische Reste für $-NX_1X_2$ sind Pyrrolidino, Piperidino oder Morpholino.

Die Substituenten $X_1$ und $X_2$ sind vorzugsweise Cyclohexyl, Tolyl, Benzyl, Phenyl, Cyano-Niederalkyl, z.B. 2-Cyanoethyl oder in erster Linie Niederalkyl, wie z.B. Methyl, Ethyl, n-Propyl, Isopropyl, n-Butyl oder Isoamyl. $-NX_1X_2$ ist bevorzugt auch Pyrrolidino, N-Niederalkyl-N-tetrahydrofurfurylamino, 4-Diniederalkylaminophenylamino oder 4-(4′-Phenylamino-phenylamino)-phenylamino.

Niederalkyl, Niederalkoxy und Niederalkylthio stellen solche Gruppen oder Gruppenbestandteile dar, die 1 bis 6, insbesondere 1 bis 4 Kohlenstoffatome aufweisen. Beispiele für derartige Gruppen sind Methyl, Ethyl, n-Propyl, Isopropyl, n-Butyl, sek.Butyl, Amyl, Isoamyl oder Hexyl bzw. Methoxy, Ethoxy, Isopropoxy, Isobutoxy, tert.Butoxy oder Amyloxy bzw. Methylthio, Ethylthio, Propylthio oder Butylthio.

Halogen bedeutet beispielsweise Fluor, Brom oder vorzugsweise Chlor.

"Acyl" ist besonders Formyl, Niederalkylcarbonyl, wie z.B. Acetyl oder Propionyl, oder Benzoyl. Weitere Acylreste können Niederalkylsulfonyl, wie z.B. Methylsulfonyl oder Ethylsulfonyl, Niederalkoxysulfonyl, wie z.B. Methoxysulfonyl oder Ethoxysulfonyl, sowie Phenylsulfonyl oder Phenoxysulfonyl sein. Benzoyl und Phenylsulfonyl können durch Halogen, Methyl, Methoxy oder Ethoxy substituiert sein.

Hervorzuheben sich die Lactamverbindungen der Formel (1), worin

A einen gegebenenfalls durch Halogen, Cyano, Nitro, $C_1$-$C_4$-Alkyl, $C_1$-$C_4$-Alkoxy, $C_1$-$C_4$-Alkylthio, $C_1$-$C_4$-Alkylcarbonyl, $C_1$-$C_4$-Alkoxycarbonyl, Amino, $C_1$-$C_4$-Alkylamino, Di-$C_1$-$C_4$-Alkylamino oder $C_1$-$C_4$-Alkylcarbonylamino substituierten Benzolring;

B einen unsubstituierten oder durch Halogen, $C_1$-$C_4$-Alkyl, $C_1$-$C_4$-Alkoxy, Benzyloxy, $C_1$-$C_4$-Alkylcarbonylamino, Mono- oder Di-$C_1$-$C_4$-Alkylamino substituierten Benzolkern;

Z $-\underset{|}{N}R$, O oder S;

R Wasserstoff; unsubstituiertes oder durch Halogen, Cyano, Benzoyl, $C_1$-$C_4$-Alkylcarbonyl, Phenoxysulfonyl, Phenylsulfonyl, $C_1$-$C_4$-Alkylsulfonyl, Di-$C_1$-$C_4$-Alkylamino oder $C_1$-$C_4$-Alkoxy substituiertes $C_1$-$C_4$-Alkyl; unsubstituiertes oder durch Halogen, Nitro oder Trifluormethyl, ringsubstituiertes Phenyl oder Benzyl; $C_1$-$C_4$-Alkyl-carbonyl; $C_1$-$C_4$-Alkyl-$SO_2$; $C_1$-$C_4$-Alkoxy-$SO_2$; N-$C_1$-$C_4$-alkylcarbamoyl; oder gegebenenfalls durch $C_1$-

$C_4$-Alkyl, Halogen, $C_1$-$C_4$-Alkoxy, $C_1$-$C_4$-Halogenalkyl, Nitro, oder Cyano ringsubstituiertes N-Phenylcarbamoyl;

Q gegebenenfalls bis zu zweifach durch $C_1$-$C_4$-Alkyl, Phenyl, $C_5$-$C_6$-Spiroalkyl substituiertes $C_2$-$C_3$-Alkylen; 1,2-Cyclohexylen; 1,2-Cyclopentylen; 1,2-Phenylen; 1,8-Naphthylen; 1,2-Naphthylen; 1,3-Naphthylen; oder

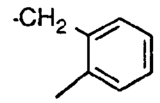 ;

und

$X_1$ und $X_2$, unabhängig voneinander, je Wasserstoff; unsubstituiertes oder durch $C_1$-$C_4$-Alkoxy, Hydroxy oder Cyano, substituiertes $C_1$-$C_4$-Alkyl; $C_1$-$C_4$-Alkylcarbonyl; oder ($X_1$ und $X_2$) zusammen mit dem gemeinsamen Stickstoffatom einen fünf- oder sechsgliedrigen gesättigten heterocyclischen Rest bedeuten.

Besonders wichtige Lactamverbindungen entsprechen der Formel

(2) 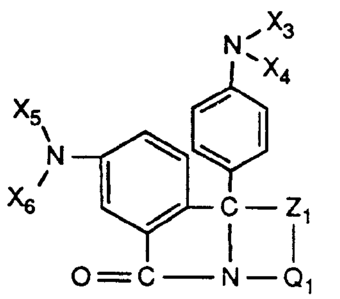

worin

$X_3$ und $X_4$ und $X_5$ und $X_5$ gleiche oder voneinander verschiedene Niederalkyl- oder Phenylreste darstellen; oder

$X_3$ und $X_4$ gemeinsam mit dem Stickstoffatom, an welches sie gebunden sind, Pyrrolidin;

$Q_1$ $C_2$-$C_3$-Alkylen, 1,2-Cyclohexylen, 1,2-Phenylen oder 1,8-Naphthylen;

$Z_1$ -O-, -S- oder $-\underset{|}{N}R_1$ ;

und

$R_1$ Wasserstoff, Phenyl, Benzyl, $C_1$-$C_4$-Alkyl, Cyano-$C_1$-$C_4$-Alkyl, $C_1$-$C_4$-Alkylcarbonyl, N-$C_1$-$C_4$-Alkylcarbamoyl oder gegebenenfalls durch Halogen, Nitro, Trifluormethyl, Niederalkyl oder Niederalkoxy substituiertes N-Phenylcarbamoyl bedeuten.

Unter den Lactamverbindungen der Formel (2) sind die Verbindungen, in denen die X-Reste identisch sind und Niederalkyl, wie n-Butyl, Ethyl und besonders Methyl bedeuten, bevorzugt. $Q_1$ ist vorzugsweise $C_2$-$C_3$-Alkylen oder 1,2-Phenylen. $Z_1$ ist vorsugsweise

$$-\underset{|}{N}R_1$$

und $R_1$ ist besonders Wasserstoff, Phenyl, Niederalkyl oder Cyano-Niederalkyl.

Von besonderem Interesse sind Lactamverbindungen der Formel

(3) 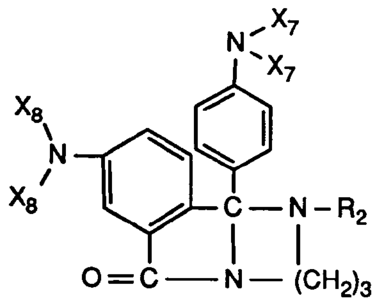

worin

$X_7$ und $X_8$, unabhängig voneinander, $C_1$-$C_4$-Alkyl und besonders Methyl und $R_2$ Wasserstoff, Phenyl, $C_1$-$C_4$-Alkyl oder Cyano-Niederalkyl bedeuten.

Die erfindungsgemässen Lactamverbindungen der Formeln (1) bis (3) stellen neue Verbindungen dar und können nach an sich bekannten Methoden hergestellt werden.

Vorteilhafterweise werden die erfindungsgemässen Lactamverbindungen dadurch hergestellt, dass man eine Ketosäure der Formel

(4) 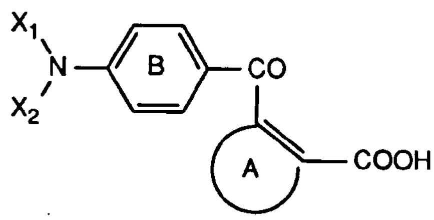

mit einer bifunktionellen Aminoverbindung der Formel

(5) $H_2N$-Q-Z-H

umsetzt, worin in den Formeln (4) und (5) A, B, $X_1$, $X_2$, Q und Z die angegebene Bedeutung haben.

Zweckmässig arbeitet man in einem nicht an der Kondensation teilnehmenden organischen Lösungsmittel und unter Rückfluss.

Geeignete organische Lösungsmittel, die das Reaktionsmedium bilden, sind cycloaliphatische oder vorzugsweise aromatische Kohlenwasserstoffe, wie z.B. Cyclohexan, Benzol, Toluol oder Xylol; Chlorkohlenwasserstoffe, wie z.B. Ethylenchlorid, Tetrachlorethylen oder Chlorbenzole, wie z.B. Chlorbenzol, Chlortoluol oder Dichlorbenzol; cyclische Ether, wie z.B. Dioxan oder Tetrahydrofuran; Dimethylsulfoxid oder Nitrile aliphatischer Monocarbonsäuren, wie z.B. Acetonitril, Propionitril oder Butyronitril. Auch Mischungen der genannten Lösungsmittel können verwendet werden. Bevorzugte Lösungsmittel sind Chlorbenzol, Chlortoluol und besonders Toluol.

Die Isolierung des Endproduktes erfolgt in allgemein bekannter Weise durch Trennung der gebildeten Wasserphase und Entfernung des Lösungsmittels oder durch Behandeln mit geeigneten organischen Lösungsmitteln, wie z.B. Methanol, Isopropanol oder Petrolether.

Die Ausgangsstoffe der Formel (4) sind z.B. in den Offenlegungsschriften DE-A-1 795 737, DE-A-2 709 580, GB-A-1 443 617 und US-A-4 062 866 beschrieben.

Die bifunktionellen Verbindungen der Formel (5) können je nach der Bedeutung von Z als Diamine, Aminoalkohole oder Mercaptoamine eingesetzt werden.

Geeignete Aminoverbindungen der Formel (5) zur Umsetzung mit den Ketosäuren der Formel (4) sind 1,2-Ethylendiamin, 1,2-Propylendiamin, 1,3-Propylendiamin, 1,2-Phenylendiamin, 1,2-Xylendiamin, 1,8-Naphthalindiamin, 2,2′-Diaminodiphenyl, 1,2-Cyclohexylendiamin, 2-Mercaptoethylamin, 2-Hydroxyethylamin, 2-Mercaptopropylamin, 3-Mercaptopropylamin, 3-Hydroxypropylamin, 1-Amino-2-hydroxy-cyclohexan, 2-Aminophenol, 2-Aminothiophenol, 2-Aminobenzylalkohol, 2-Amino-1-butanol, 1-Amino-2-butanol, 2-Amino-1-phenylethanol, 2-Amino-1-phenyl-1-propanol, 2,2-Dimethyl-1,3-propylendiamin, 3-Amino-2,2-dimethyl-1-propanol, 2-Amino-1,3-propandiol, N-Methylethylendiamin, N-Methyl- 1,3-propylendiamin, N-n-Butyl-1,3-propylendiamin, N-tert.Butyl- 1,3-propylendiamin, N-2-Cyanoethylethylendiamin, N-2′-Cyanoethyl-1,3-propylendiamin, N-Cyanoisopropylethylendiamin, N-Cyanoisopropyl- 1,3-propylendiamin, N-Phenylethylendiamin, N-Phenyl- 1,3-propylendiamin, N-Benzylethylendiamin, N-Benzyl- 1,3-propylendiamin, N-Cyclohexyl- 1,3-propylendiamin, 1-Aminomethyl-cyclohexanol, 2-(2′-Aminoethyl)-phenol, N′-[3-Aminopropyl]-N,N-dimethylhydrazin, 2-(2′-Hydroxyethyl)anilin, o-Aminomethylphenol, 1-Aminomethyl-1-cyclohexanol.

Verbindungen der Formel (1), worin

Z -NR

und R Acyl, N-Niederalkylcarbamoyl oder gegebenenfalls substituiertes N-Phenylcarbamoyl bedeuten, können auch dadurch hergestellt werden, dass man eine Lactamverbindung der Formel (1), worin Z -NH- bedeutet, auf übliche Weise mit einem reaktiven funktionellen Derivat einer Carbonsäure oder einer Sulfonsäure, besonders Halogeniden oder Anhydriden z.B.Acetanhydrid, Acetylchlorid, Acetylbromid, Benzoylchlorid, Benzolsulfonylchlorid oder auch mit Isocyanaten, wie z.B. Niederalkylisocyanaten, Phenylisocyanat, Halogenphenylisocyanat oder Niederalkylphenylisocyanat umsetzt.

Die Lactamverbindungen der Formeln (1) bis (3) sind normalerweise farblos oder höchstens schwach gefärbt. Wenn diese Farbbildner mit einem vorzugsweise sauren Entwickler, d.h. einem Elektronenakzeptor, in Kontakt gebracht werden, so ergeben sie je nach der Bedeutung von Z und dem verwendeten Entwickler intensive gelbe, rote, violette, grün-blaue, blaue oder grüne Farbtöne, die sublimations- und lichtecht sind. Die Lactame der Formeln (1) bis (3) sind auch sehr wertvoll im Gemisch mit einem oder mehreren anderen bekannten Farbbildnern, z.B. 3,3-(Bis-aminophenyl-)-phthaliden, wie CVL, 3-Indolyl-3-aminophenyl-aza- oder -diazaphthaliden, (3,3-Bis-indolyl-)-phthaliden, 3-Aminofluoranen, 2,6-Diaminofluoranen, 2,6-Diamino-3-methylfluoranen, 3,6-Bis-alkoxyfluoranen, 3,6-Bisdiarylaminofluoranen, Leukoauraminen, Spiropyranen, Spirodipyranen, Chromenopyrazolen, Chromenoindolen, Phenoxazinen, Phenothiazinen, Chinazolinen, Rhodaminlaktamen, Carbazolylmethanen oder weiteren Triarylmethan-leukofarbstoffen, um marine-blaue, graue oder schwarze Färbungen zu ergeben.

Die Lactamverbindungen der Formeln (1) bis (3) zeigen sowohl auf aktivierten Tonen, wie auch auf phenolischen Unterlagen eine ausgezeichnete Farbintensität und Lichtechtheit. Sie eignen sich vor allem als Farbbildner für die Verwendung in einem wärmeempfindlichen oder insbesondere druckempfindlichen Aufzeichnungsmaterial, das sowohl Kopier- als auch Registriermaterial sein kann. Sie zeichnen sich dadurch aus, dass sie pH stabil und in den Kapselölen hervorragend löslich sind. Nach Belichtung in CB-Blatt weisen sie eine geringe Abnahme der Farbstärke (CB-Desaktivierung) auf.

Ein druckempfindliches Material besteht beispielsweise aus mindestens einem Paar von Blättern, die mindestens einen Farbbildner der Formeln (1) bis (3) gelöst in einem organischen Lösungsmittel und einen Elektronenakzeptor als Entwickler enthalten.

Typische Beispiele für solche Entwickler sind Aktivton-Substanzen, wie Attapulgus-Ton, Säureton, Bentonit, Montmorillonit, aktivierter Ton, wie z.B. säureaktiviertes Bentonit oder Montmorillonit, ferner Zeolith, Halloysit, Siliciumdioxyd, Aluminiumoxid, Aluminiumsulfat, Aluminiumphosphat, Zinkchlorid, Zinknitrat, Zirkondioxid, aktiviertes Kaolin oder irgendein beliebiger Ton. Als Entwickler können auch sauer reagierende, organische Verbindungen, wie z.B. gegebenenfalls ringsubstituierte Phenole, Resorcine, Salicylsäuren, wie z.B. 3,5-Bis-($\alpha$,$\alpha$-dimethylbenzyl-)-salicylsäure oder 3,5-Bis-($\alpha$-methylbenzyl)-salicylsäure oder Salicylsäureester und deren Metallsalze, z.B. Zinksalze, sowie ein sauer reagierendes, polymeres Material, wie z.B. ein phenolisches Polymerisat, ein Alkylphenolacetylenharz, ein Maleinsäure-Kolophonium-Harz oder ein teilweise oder vollständig hydrolysiertes Polymerisat von Maleinsäureanhydrid mit Styrol, Ethylen oder Vinylmethylether, oder Carboxymethylen verwendet werden. Es können auch Mischungen der genannten monomeren und polymeren Verbindungen eingesetzt werden. Besonders bevorzugte Entwickler sind säureaktiviertes Bentonit, Zinksalicylate, wie z.B. Zink-3,5-bis-$\alpha$-methylbenzylsalicylat oder die Kondensationsprodukte von p-substituierten Phenolen mit Formaldehyd. Die letzteren können auch mit Zink modifiziert sein. Die Zinksalicylate sind z.B. in EP-A-181 283 oder DE-A-2 242 250 beschrieben.

Die Entwickler können zusätzlich auch im Gemisch mit an sich unreaktiven oder wenig reaktiven Pigmenten oder weiteren Hilfsstoffen wie Kieselgel oder UV-Absorbern, wie z.B. 2-(2′-Hydroxyphenyl-)benztriazolen oder 2-Hydroxyphenyl- 1,2,3-triazinen eingesetzt werden. Beispiele für solche Pigmente sind: Talk, Titandioxid, Aluminiumoxid, Aluminiumhydroxyd, Zinkoxid, Kreide, Tone wie Kaolin, sowie organische Pigmente, z.B. Harnstoff-Formaldehydkondensate (BET-Oberfläche 2-75 $m^2/g$) oder Melamin-Formaldehyd-Kondensationsprodukte.

Der Farbbildner liefert an den Punkten, an denen er mit dem Elektronenakzeptor in Kontakt kommt, eine gefärbte Markierung. Um eine frühzeitige Aktivierung der in dem druckempfindlichen Aufzeichnungsmaterial vorhandenen Farbbildner zu verhindern, werden diese in der Regel von dem Elektronenakzeptor getrennt. Dies kann zweckmässig erzielt werden, indem man die Farbbildner in schaum-, schwamm- oder bienenwabenartigen Strukturen einarbeitet. Vorzugsweise sind die Farbbildner in Mikrokapseln eingeschlossen, die sich in der Regel durch Druck zerbrechen lassen.

Beim Zerbrechen der Kapseln durch Druck, beispielsweise mittels eines Bleistiftes, wird die Farbbildnerlösung auf ein benachbartes mit einem Elektronenakzeptor beschichtetes Blatt übertragen, wodurch eine farbige Stelle erzeugt wird. Die Farbe resultiert aus dem dabei gebildeten Farbstoff, der im sichtbaren Bereich des elektromagnetischen Spektrums absorbiert.

Die Farbbildner werden vorzugsweise in Form von Lösungen in organischen Lösungsmittel eingekapselt. Beispiele für geeignete Lösungsmittel sind vorzugsweise nichtflüchtige Lösungsmittel, z.B. halogeniertes Paraffin, Benzol oder Diphenyl, wie Chlorparaffin, Trichlorbenzol, Monochlordiphenyl, Dichlordiphenyl oder Trichlordiphenyl, Ester, wie z.B. Tricresylphosphat, Di-n-butylphthalat, Dioctylphthalat, Trichlorethylphosphat, aromatische Ether, wie Benzylphenylether, Kohlenwasserstofföle, wie Paraffin oder Kerosin, aromatische Kohlenwasserstoffe, z.B. mit Isopropyl, Isobutyl, sek-Butyl oder tert.-Butyl alkylierte Derivate von Diphenyl, Naphthalin oder Terphenyl, Dibenzyltoluol, partiell hydriertes Terphenyl, mono- bis tetra-$C_1$-$C_3$-alkylierte Diphenylalkane, Dodecylbenzol, benzylierte Xylole, Phenylxylylethan oder weitere chlorierte oder hydrierte, kondensierte, aromatische Kohlenwasserstoffe. Oft werden Mischungen verschiedener Lösungsmittel, insbesondere Mischungen aus Paraffinölen oder Kerosin und Diisopropylnaphthalin oder partiell hydriertem Terphenyl, eingesetzt, um eine optimale Löslichkeit für die Farbbildung, eine rasche und intensive Färbung und eine für die Mikroverkapselung günstige Viskosität zu erreichen. Bei der Einkapselung zeichnen sich die erfindungsgemässen Lactame dadurch aus, dass sie gut löslich sind und pH-Beständigkeit z.B. in einem pH-Bereich von 4 bis 10 zeigen.

Die Kapselwände können durch Koazervationskräfte gleichmässig um die Tröpfchen der Farbbildnerlösung herum gebildet werden, wobei das Einkapselungsmaterial z.B. in der US-Patentschrift 2,800,457 beschrieben ist. Die Kapsel können vorzugsweise auch aus einem Aminoplast oder modifizierten Aminoplasten durch Polykondensation gebildet werden, wie es in den britischen Patentschriften 989,264, 1,156,725, 1,301,052, 4,100,103 und 1,355,124 beschrieben ist. Ebenfalls geeignet sind Mikrokapseln, welche durch Grenzflächenpolymerisation gebildet werden, wie z.B. Kapseln aus Polyester, Polycarbonat, Polysulfonamid, Polysulfonat, besonders aber aus Polyamid, Polyharnstoff oder Polyurethan.

Die Farbbildner der Formel (1) bis (3) enthaltenden Mikrokapseln können zur Herstellung von druckempfindlichen Kopiermaterialien der verschiedensten bekannten Arten verwendet werden. Die verschiedenen Systeme unterscheiden sich im wesentlichen voneinander durch die Anordnung der Kapseln, der Farbreaktanten und durch das Trägermaterial.

Bevorzugt wird eine Anordnung, bei der der eingekapselte Farbbildner in Form einer Schicht auf der Rückseite eines Uebertragungsblattes und der Elektronenakzeptor (Farbentwickler) in Form einer Schicht auf der Vorderseite eines Empfangsblattes vorhanden sind. Eine andere Anordnung der Bestandteile besteht darin, dass die den Farbbildner enthaltenden Mikrokapseln und der Entwickler in oder auf dem gleichen Blatt in Form einer oder mehrerer Einzelschichten vorliegen oder der Entwickler im Trägermaterial eingebaut ist.

Die Kapseln werden vorzugsweise mittels eines geeigneten Binders auf dem Träger befestigt. Da Papier das bevorzugte Trägermaterial ist, handelt es sich bei diesem Binder hauptsächlich um Papierbeschichtungsmittel, wie Gummi arabicum, Polyvinylalkohol, Hydroxymethylcellulose, Casein, Methylcellulose, Dextrin, Stärke, Stärkederivate oder Polymerlatices. Letztere sind beispielsweise Butadien-Styrolcopolymerisate oder Acrylhomo- oder -copolymere.

Als Papier werden nicht nur normale Papiere aus Cellulosefasern, sondern auch Papiere, in denen die Cellulosefasern (teilweise oder vollständig) durch Fasern aus synthetischen Polymerisaten ersetzt sind, verwendet. Schichtträger kann auch eine Kunststoffolie sein.

Vorzugsweise besteht das Durchschreibematerial auch darin, dass es eine kapselfreie, den Farbbildner enthaltende Schicht und eine farbentwickelnde Schicht, die als Farbentwickler mindestens ein anorganisches Metallsalz eines mehrwertigen Metalles, vor allem Halogenide oder Nitrate, wie z.B. Zinkchlorid, Zinnchlorid, Zinknitrat oder deren Gemische enthält, aufweist.

Die Verbindungen der Formeln (1) bis (3) können auch als Farbbildner in einem thermoreaktiven Aufzeichnungsmaterial verwendet werden. Dieses enthält in der Regel mindestens einen Schichtträger, einen oder mehrere Farbbildner, und einen Elektronenakzeptor und gegebenenfalls auch ein Bindemittel und/oder Wachs. Gewünschtenfalls können auch Aktivatoren oder Sensibilisatoren, z.B. Benzyldiphenyl, im Aufzeichnungsmaterial vorhanden sein.

Thermoreaktive Aufzeichnungssysteme umfassen, z.B. wärmeempfindliche Aufzeichnungs- und Kopiermaterialien und -papiere. Diese Systeme werden beispielsweise zum Aufzeichnen von Informationen, z.B. in elektronischen Rechnern, Ferndruckern, Fernschreibern oder in Aufzeichnungsgeräten und Messinstrumenten, wie z.B. Elektrocardiographen, verwendet. Die Bilderzeugung (Markierung) kann auch manuell mit einer erhitzten Feder erfolgen. Eine weitere Einrichtung zur Erzeugung von Markierungen mittels Wärme sind Laserstrahlen.

Das thermoreaktive Aufzeichnungsmaterial kann so aufgebaut sein, dass der Farbbildner in einer Bindemittelschicht gelöst oder dispergiert ist und in einer zweiten Schicht der Entwickler in dem Bindemittel gelöst

und dispergiert ist. Eine andere Möglichkeit besteht darin, dass sowohl der Farbbildner als auch der Entwickler in einer Schicht dispergiert sind. Die Schicht bzw. Schichten werden in spezifischen Bezirken mittels Wärme erweicht, worauf sich sofort in den erwärmten Teilen die erwünschte Farbe entwickelt.

Als Entwickler eignen sich die gleichen Elektronenakzeptoren, wie sie in druckempfindlichen Papieren verwendet werden. Beispiele für Entwickler sind die bereits erwähnten Tonminerale und Phenolharze, oder auch phenolische Verbindungen, wie sie beispielsweise in der DE-PS 1 251 348 beschrieben sind, z.B. 4-tert.-Butylphenol, 4-Phenylphenol, Methylen-bis-(p-phenylphenol), 4-Hydroxydiphenylether, α-Naphthol, β-Naphthol, 4-Hydroxybenzoesäure-methylester oder -benzylester, 4-Hydroxydiphenylsulfon, 4′-Hydroxy-4-methyldiphenylsulfon, 4′-Hydroxy-4-isopropoxydiphenylsulfon, 4,4′-Cyclohexylidendiphenol, 4,4′-Isopropylidendiphenol, 4,4′-Isopropyliden-bis-(2-methylphenol), ein Antipyrinkomplex von Zinkthiocyanat, ein Pyridinkomplex von Zinkthiocyanat, 4,4-Bis-(4-hydroxyphenyl)valeriansäure, Hydrochinon, Pyrogallol, Phloroglucin, p-, m-, o-Hydroxybenzoesäure, Hydroxyphthalsäure, Gallussäure, 1-Hydroxy-2-naphthoesäure sowie Borsäure oder organische, vorzugsweise aliphatische Dicarbonsäuren, wie z.B. Weinsäure, Oxalsäure, Maleinsäure, Zitronensäure, Citraconsäure oder Bernsteinsäure.

Vorzugsweise werden zur Herstellung des thermoreaktiven Aufzeichnungsmaterials schmelzbare, filmbildende Bindemittel verwendet. Diese Bindemittel sind normalerweise wasserlöslich, während die Lactame und der Entwickler in Wasser schwer löslich oder unlöslich sind. Das Bindemittel sollte in der Lage sein, den Farbbildner und den Entwickler bei Raumtemperatur zu dispergieren und zu fixieren.

Bei Einwirkung von Wärme erweicht oder schmilzt das Bindemittel, so dass der Farbbildner mit dem Entwickler in Kontakt kommt und sich eine Farbe bilden kann. Wasserlösliche oder mindestens in Wasser quellbare Bindemittel sind z.B. hydrophile Polymerisate, wie Polyvinylalkohol, Polyacrylsäure, Hydroxyethylcellulose, Methylcellulose, Carboxymethylcellulose, Polyacrylamid, Polyvinylpyrrolidon, Gelatine, Stärke oder veretherte Maisstärke.

Wenn der Farbbildner und der Entwickler in zwei getrennten Schichten vorliegen, können in Wasser unlösliche Bindemittel, d.h. in nichtpolaren oder nur schwach polaren Lösungsmitteln lösliche Bindemittel, wie z.B. Naturkautschuk, synthetischer Kautschuk, chlorierter Kautschuk, Alkydharze, Polystyrol, Styrol/Butadien-Mischpolymerisate, Polymethylacrylate, Ethylcellulose, Nitrocellulose und Polyvinylcarbazol, verwendet werden. Die bevorzugte Anordnung ist jedoch diejenige, bei der der Farbbildner und der Entwickler in einer Schicht in einem wasserlöslichen Bindemittel enthalten sind.

Um die Stabilität des wärmeempfindlichen Aufzeichnungsmaterials oder die Bilddichte des entwickelten Bildes zu gewährleisten, kann das Material mit einer zusätzlichen Schutzschicht versehen sein. Derartige Schutzschichten bestehen in der Regel aus wasserlöslichen und/oder wasserunlöslichen Harzen, die herkömmliche Polymermaterialien oder wässrige Emulsionen von diesen Polymermaterialien sind.

Die thermoreaktiven Schichten und Harzschichten können weitere Zusätze enthalten. Zur Verbesserung des Weissgrades, zur Erleichterung des Bedruckens der Papiere und zur Verhinderung des Festklebens der erhitzten Feder können diese Schichten, z.B. Talk, Titandioxyd, Zinkoxyd, Aluminiumhydroxyd, Calciumcarbonat (z.B. Kreide), Tone oder auch organische Pigmente, wie z.B. Harnstoff-Formaldehydpolymerisate enthalten. Um zu bewirken, dass nur innnerhalb eines begrenzten Temperaturbereiches die Farbe gebildet wird, können Substanzen, wie Harnstoff, Thioharnstoff, Diphenylthioharnstoff, Acetamid, Acetanilid, Benzolsulfanilid, Stearinsäureamid, Bis-stearoylethylendiamid, Phthalsäureanhydrid, Metallstearate, wie z.B. Zinkstearat, Phthalsäurenitril, Dimethylterephthalat, Dibenzylterephthalat oder andere entsprechende, schmelzbare Produkte, welche das gleichzeitige Schmelzen des Farbbildners und des Entwicklers induzieren, zugesetzt werden. Bevorzugt enthalten thermographische Aufzeichnungsmaterialien Wachse, z.B. Carnaubawachs, Montanwachs, Paraffinwachs, Polyethylenwachs, Kondensate höherer Fettsäureamide und Formaldehyde und Kondensate höherer Fettsäuren und Ethylendiamin.

Eine weitere Anwendung der Verbindungen der Formeln (1) bis (3) ist die Herstellung eines Farbbildes mittels photohärtbarer Mikrokapseln, wie sie z.B. in der DE-OS 3 247 488 beschrieben sind.

In den folgenden Beispielen beziehen sich die angegebenen Prozentsätze, wenn nichts anderes angegeben ist, auf das Gewicht.

Beispiel 1:

15,6 g 2-(4′-Dimethylaminobenzoyl)-5-Dimethylamino-benzoesäure werden in 100 ml Toluol angeschlämmt und mit 7,4 g 1,3-Propylendiamin versetzt. Das Reaktionsgemisch wird solange am Rückfluss erhitzt, bis die entstehende Wasserphase konstant bleibt. Durch Zusetzen von Petrolether und Stehenlassen kristallisiert das Produkt aus. Nach Abtrennen und Trocknen erhält man 17 g einer Lactamverbindung der Formel

(11)

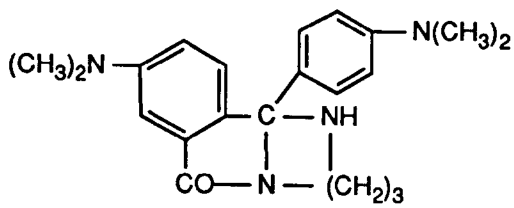

mit einem Schmelzpunkt von 191-193°C.

Beispiel 2:

Verwendet man in Beispiel 1 32 g 2-(4'-Dimethylaminobenzoyl)-5-dimethylaminobenzoesäure und 12 g Ethylendiamin anstelle von 1,3-Propylendiamin, so erhält man 30,6 g einer Lactamverbindung der Formel

(12)

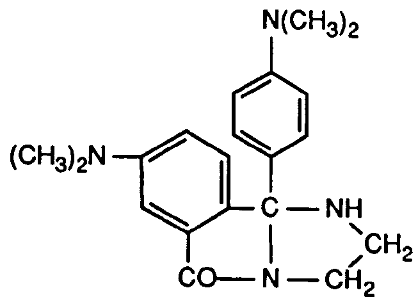

mit einem Schmelzpunkt (Smp.) von 178- 179°C.

Beispiel 3:

Verwendet man in Beispiel 1 anstelle von 1,3-Propylendiamin 8,8 g 3-Methylaminopropylamin in 130 ml Toluol und 0,1 g p-Toluolsulfonsäure, so erhält man eine Lactamverbindung der Formel

(13)

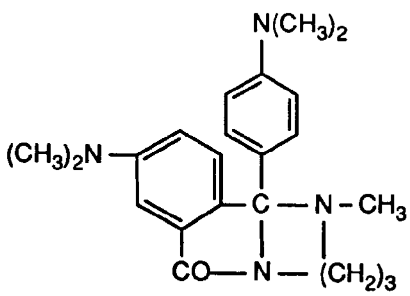

Smp. 145-148°C

Beispiel 4:

Verwendet man in Beispiel 1 anstelle von 1,3-Propylendiamin 6,1 g Ethanolamin in 100 ml Toluol und verfährt ansonst wie im Beispiel beschrieben, so erhält man nach Chromatographie 6 g einer Lactamverbindung der Formel

(14) 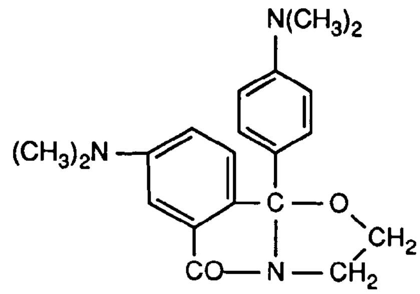

Beispiel 5:

Verwendet man in Beispiel 1 anstelle von 1,3-Propylendiamin 4,62 g Cysteamin und verfährt ansonst wie im Beispiel beschrieben, so erhält man die Lactamverbindung der Formel

(15) 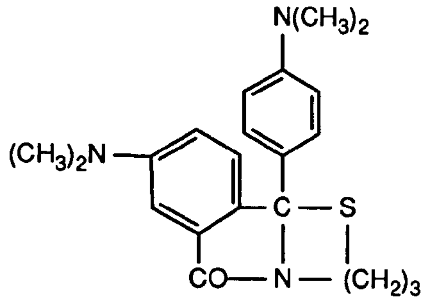

als gelbliches Oel.

Beispiel 6:

Verwendet man in Beispiel 1 2,4 g Phenylendiamin anstelle von 1,3-Propylendiamin und verfährt ansonst wie im Beispiel beschrieben, so erhält man die Lactamverbindung der Formel

(16) 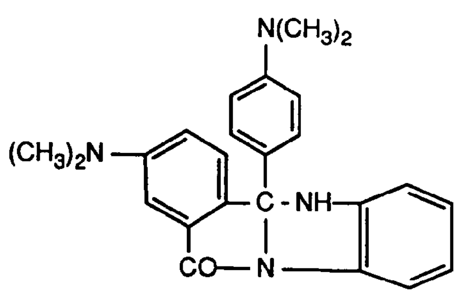

mit einem Schmelzpunkt von 266-268°C.

Beispiel 7:

Verwendet man in Beispiel 1 anstelle von 1,3-Propylendiamin 7,5 g 3-Amino-1-propanol unter Zusatz von 0,1 g p-Toluolsulfonsäure und verfähren ansonst wie im Beispiel beschrieben, so erhält man 12,1 g einer Lactamverbindung der Formel

(17) 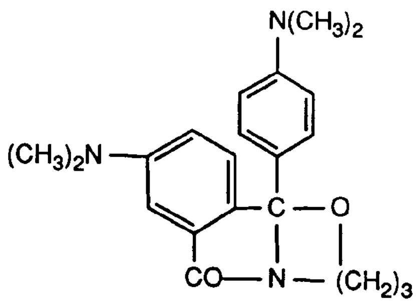

mit einem Schmelzpunkt von 143-146°C.

Beispiel 8:

13,3 g 2-(4′-Dimethylaminobenzoyl)-benzoesäure werden in 150 ml Toluol angeschlämmt und mit 6 g Ethylendiamin versetzt. Das Reaktionsgemisch wird solange am Rückfluss erhitzt, bis die entstehende Wasserphase konstant bleibt. Durch Zusetzen von Petrolether und Stehenlassen kristallisiert das Produkt aus. Nach Abtrennen und Trocknen erhält man 12,2 g einer Lactamverbindung der Formel

(18) 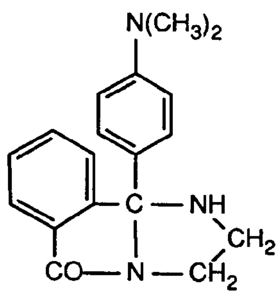 Smp. 224-225°C

Beispiel 9:

Verwendet man in Beispiel 8 anstelle der dort angegebenen Ausgangsprodukte 7,5 g 2-(4′-Di-n-butylamino-2′-ethoxybenzoyl)-benzoesäure und 2,3 g Ethylendiamin, so erhält man 7,32 g gelbes Oel einer Lactamverbindung der Formel

(19) 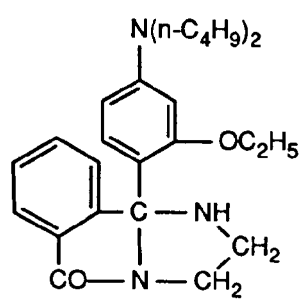

Beispiel 10:

Verwendet man in Beispiel 8 anstelle von Ethylendiamin 3,66 g Ethanolamin, so erhält man eine Lactamverbindung der Formel

(20)

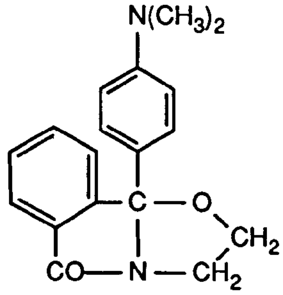

mit einem Schmelzpunkt von 89-90°C.

Beispiel 11:

Verwendet man in Beispiel 8 anstelle von Ethylendiamin 4,62 g Cysteamin und verfährt ansonst wie im Beispiel beschrieben, so erhält man nach Chromatographie 9,19 g einer Lactamverbindung der Formel

(21)

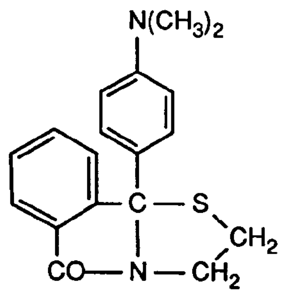

Beispiel 12:

4,4 g 2-(4'-Dimethylaminobenzoyl)-5-dimethylaminobenzoesäure werden in 150 ml Toluol angeschlämmt und unter Zusatz von 0,3 g p-Toluolsulfonsäure mit 2,2 g 2,2-Dimethyl-1,3-propylendiamin reagieren gelassen. Man erhält 2,9 g einer Lactamverbindung der Formel

(22)

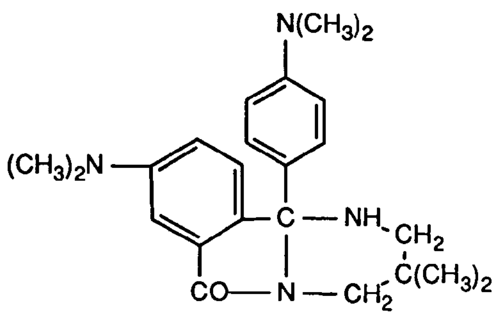

mit einem Schmelzpunkt von 221-223°C (Zers.).

Beispiel 13:

8,9 g 2-(4'-Diethylaminobenzoyl)-5-dimethylaminobenzoesäure werden in 200 ml Toluol angeschlämmt und mit 3,8 g 1,3-Propylendiamin wie in Beispiel 1 beschrieben, reagieren gelassen. Man erhält 6,9 g einer Lactamverbindung der Formel

(23)

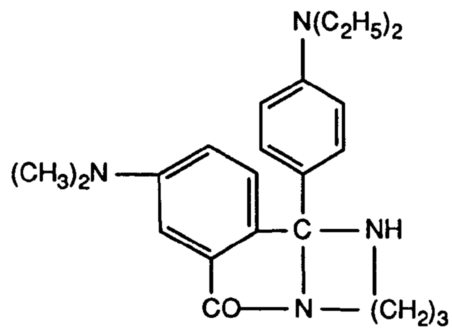

mit einem Schmelzpunkt von 161-163°C.

Beispiel 14:

4,4 g 2-(4'-Dimethylaminobenzoyl)-5-dimethylaminobenzoesäure werden wie in Beispiel 1 beschrieben mit 2,9 g (±) -2-Amino-1-phenylethanol umgesetzt. Man erhält 4,32 g eines 2:1-Diastereomerengemisches der Lactamverbindung der Formel

(24)

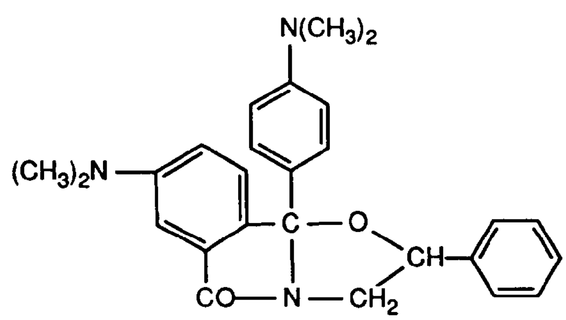

mit einem Schmelzpunkt von 233-235°C.

Beispiel 15:

Verwendet man in Beispiel 14 anstelle von ( ±) -2-Amino-1-phenylethanol 1,6 g 1-Amino-2-propanol und verfährt ansonst wie im Beispiel beschrieben, so erhält man 4,9 g des 2:1-Diastereomerengemisches der Formel

(25)

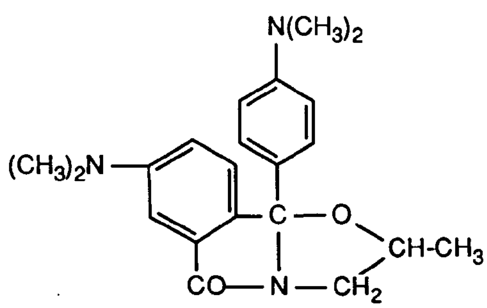

mit einem Schmelzpunkt von 62-65°C.

Beispiel 16:

10,3 g der gemäss Beispiel 8 hergestellten Lactamverbindung der Formel (18) 35 ml Acetananhydrid und 3 Tropfen Bortrifluoridetherat werden zum Sieden erhitzt. Nach dem Erkalten erhält man 6,7 g einer Lactamverbindung der Formel

(26) 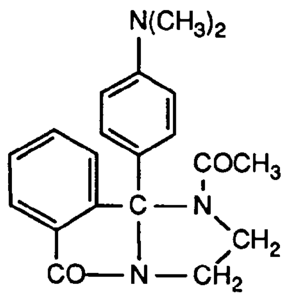 Smp. 79-82°C

Beispiel 17:

Verwendet man in Beispiel 16 anstelle der Lactamverbindung der Formel (18) die Lactamverbindung der Formel (11), so erhält man 1,6 g der Lactamverbindung der Formel

(27) 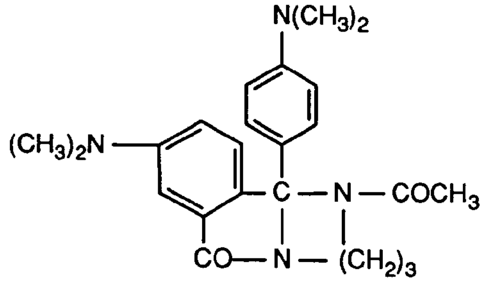 Smp. 72-78°C

Beispiel 18:

5,25 g der gemäss Beispiel 1 hergestellten Lactamverbindung der Formel (11) in 40 ml Toluol werden mit 1,8 g Phenylisocyanat in 3,5 ml Toluol langsam versetzt. Das Reaktionsgemisch wird zuerst bei Raumtemperatur gerührt und dann auf 60°C erwärmt. Hierauf wird die Toluollösung zur Trockne eingeengt und der Rückstand in Aceton aufgenommen und mit wenig n-Heptan versetzt. Man erhält 5,45 g einer Lactamverbindung der Formel

(28) 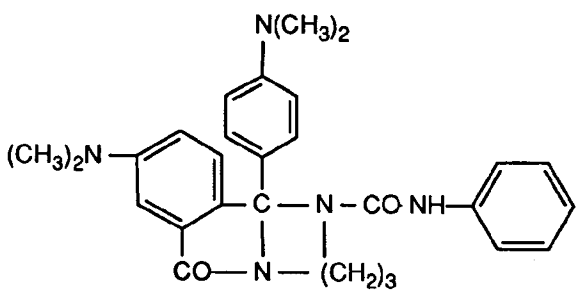 Smp. 188-190°C

Beispiel 19:

Analog Beispiel 14 erhält man aus 4,4 g 2-(4′-Dimethylamino-benzoyl)-5-dimethylaminobenzoesäure und 1,9 g R(-)-2-Aminobutanol 4,4 g des Diastereomerengemisches der Formel

(29) 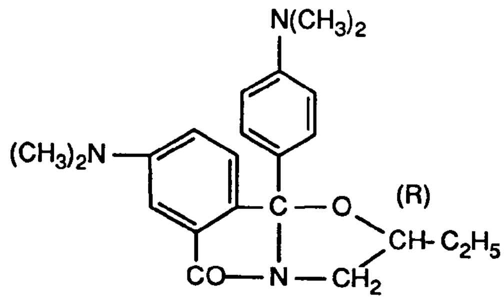 als Oel.

Beispiel 20:

Analog Beispiel 14 erhält man aus 4,4 g 2-(4'-Dimethylaminobenzoyl)-5-di-methylaminobenzoesäure und 2,6 g o-Aminomethylphenol 5,1 g der Verbindung der Formel

(30) 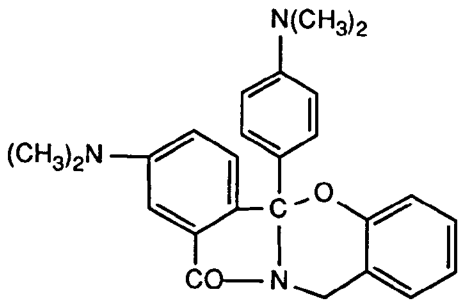 Smp. 156 - 159° C.

Beispiel 21:

Analog Beispiel 12 erhält man aus 1,6 g 2-(4'-Dimethylaminobenzoyl)-5-di-methylaminobenzoesäure und 1,0 g 1-Aminomethyl-1-cyclohexanol-hydrochlorid in 75 ml Toluol 1,9 g der Verbindung der Formel

(31) 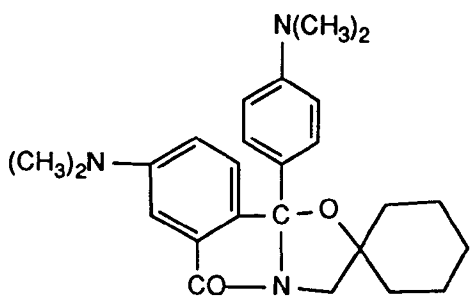 Smp. 180 - 184° C.

Beispiel 22:

Analog Beispiel 12 erhält man aus 9,4 g 2-(4'-Dimethylaminobenzoyl)-5-di-methylaminobenzoesäure und 5,0 g o-Aminophenyl-ethanol in 150 ml Toluol 2,3 g der Verbindung der Formel

(32) 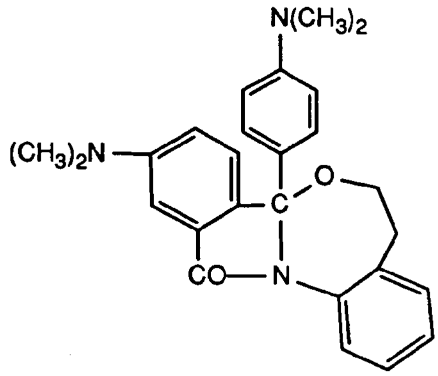 Smp. 127 - 130° C.

Beispiel 23:

5,11 g 2-(2'-Ethoxy-4-pyrrolidino-benzoyl)-benzoesäure und 1,8 g 3-Amino-1-propanol werden in 100 ml Toluol im Wasserabscheider erhitzt, bis kein Wasser mehr entsteht. Nach dem Abkühlen und Einengen fällt ein farbloses Produkt an, welches abfiltriert und mit Ethanol gewaschen wird. Man isoliert 2,5 g der Verbindung der Formel

(33) 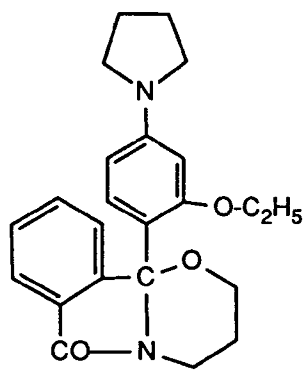 mit dem Smp. 188 - 190° C.

Beispiel 25:

Analog zu Beipiel 1 erhält man aus 11,2 g 2-(N-Methyl-N-phenylaminobenzoyl)-benzoesäure und 3,7 g Diaminopropan 7,0 g der Verbindung der Formel

(34) 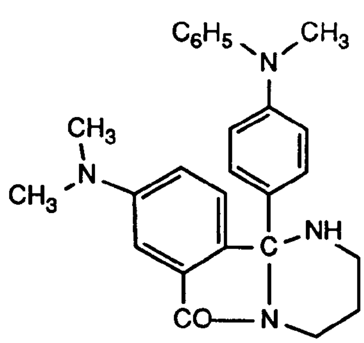 mit Smp. 202 - 206° C.

Beispiele 26-32:

Auf ähnliche Art und Weise wie im Beispiel 18 beschrieben, werden unter Verwendung des entsprechenden Isocyanats anstelle von Phenylisocyanat Lactamverbindungen der Formel (35) hergestellt

(35) 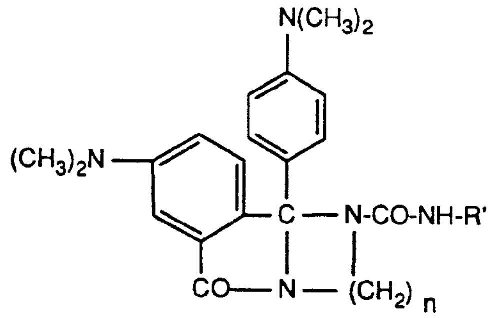

Tabelle 1:

| Bsp. | R' | n | Smp |
|---|---|---|---|
| 26 | 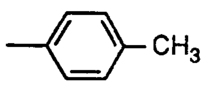 | 3 | 215-217°C |
| 27 | 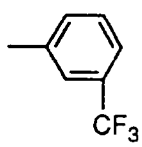 | 3 | 221°C |
| 28 | 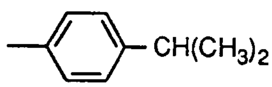 | 3 | 204-205°C |
| 29 | 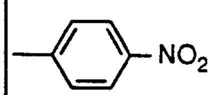 | 3 | 212°C |
| 30 | 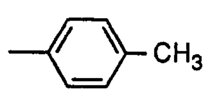 | 2 | 196-198° C |
| 31 | 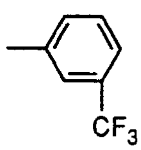 | 2 | 174-177° C |
| 32 | 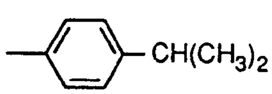 | 2 | 176-178° C |

Beispiele 33-47:

Auf ähnliche Art und Weise wie in Beispiel 1 beschrieben, werden unter Verwendung des entsprechenden N-substituierten Diamins anstelle von 1,3-Propylendiamin Lactamverbindungen der Formel (36) hergestellt

(36) 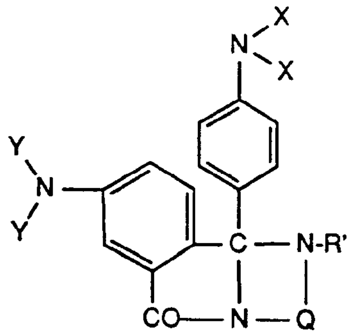

Tabelle 2:

| Bsp. | Q | R' | X | Y | Smp |
|---|---|---|---|---|---|
| 33 | $-(CH_2)_3-$ | $-n-C_4H_9$ | $CH_3$ | $CH_3$ | 124-125°C |
| 34 | $-(CH_2)_3-$ | $-C_2H_4CN$ | $CH_3$ | $CH_3$ | 161-163°C |
| 35 | $-(CH_2)_3-$ | 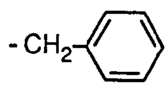 | $CH_3$ | $CH_3$ | 102-103°C |
| 36 | $-(CH_2)_3-$ | 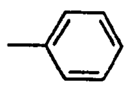 | $CH_3$ | $CH_3$ | 104-106°C |
| 37 | $-(CH_2)_3-$ | $-CH(CH_3)CH_2CN$ | $CH_3$ | $CH_3$ | 103-108°C |
| 38 | $-CH_2CH_2-$ | $-CH_3$ | $CH_3$ | $CH_3$ | 131-133°C |
| 39 | $-CH_2CH_2-$ | $-CH_2CH_2CN$ | $CH_3$ | $CH_3$ | 178-181°C |
| 40 | $-CH_2CH_2-$ | $-CH(CH_3)CH_2CN$ | $CH_3$ | $CH_3$ | 194-196°C |

Fortsetzung Tabelle 2:

| Bsp. | Q | R' | X | Y | Smp |
|---|---|---|---|---|---|
| 41 | $-CH_2CH_2-$ | 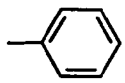 | $CH_3$ | $CH_3$ | 238-240° C |
| 42 | $-(CH_2)_3-$ | $-N(CH_3)_2$ | $CH_3$ | $CH_3$ | Oel |
| 43 | $-(CH_2)_3-$ | $-CH_3$ | $n-C_4H_9$ | $CH_3$ | 129-131° C |
| 44 | $-(CH_2)_3-$ | $-CH_2CH_2N(CH_3)_2$ | $CH_3$ | $CH_3$ | Oel |
| 45 | $-(CH_2)_3-$ | $-(CH_2)_2-OCH_2CH_3$ | $CH_3$ | $CH_3$ | Oel |
| 46 | $-(CH_2)_3-$ | $-CH_2CH_2SO_2C_6H_5$ | $CH_3$ | $CH_3$ | Oel |
| 47 | $-CH_2CH_2-$ | $-CH_2CH_2SO_3C_6H_5$ | $CH_3$ | $CH_3$ | Oel |

Beispiel 48:

Herstellung eines druckempfindlichen Kopierpapiers.

Eine Lösung von 3 g der Lactamverbindung der Formel (11) (Beispiel 1) in 80 g Diisopropylnaphthalin und 17 g Kerosin wird auf an sich bekannte Weise mit Gelatine und Carboxymethylcellulose durch Koazervation mikroverkapselt, mit Stärkelösung vermischt und auf ein Blatt Papier gestrichen. Ein zweites Blatt Papier wird auf der Frontseite mit Aktivton als Farbentwickler beschichtet. Das erste den Farbbildner enthaltende Blatt und das mit Farbentwickler beschichtete Papier werden mit den Beschichtungen benachbart aufeinandergelegt. Durch Schreiben mit der Hand oder mit der Schreibmaschine auf dem ersten Blatt wird Druck ausgeübt, und es entwickelt sich auf dem mit dem Entwickler beschichteten Blatt eine intensive grüne Kopie, die ausgezeichnet lichtecht ist.

Beispiel 33:

1 g der Lactamverbindung der Formel (11) gemäss Beispiel 1 wird in 17 g Toluol gelöst. Zu dieser Lösung gibt man unter Rühren 12 g Polyvinylacetat, 8 g Calciumcarbonat und 2 g Titandioxid. Die erhaltene Suspension wird im Gewichtsverhältnis 1: 1 mit Toluol verdünnt und mit einem 10 µm Rakel auf ein Blatt Papier gestrichen. Auf dieses Blatt Papier wird ein zweites Blatt Papier gelegt, dessen Unterseite bei einem Augtragsgewicht von 3 g/m² mit einer Mischung bestehend aus 1 Teil eines Amidwachses, 1 Teil eines Stearinwachses und 1 Teil Zinkchlorid beschichtet ist. Durch Schreiben mit der Hand oder mit der Schreibmaschine auf dem oberen Blatt wird Druck ausgeübt, und es entwickelt sich auf dem mit dem Farbbildner beschichteten Blatt eine intensive und lichtechte grüne Farbe.

## Patentansprüche

1. Chromogene Lactamverbindung der Formel

(1) 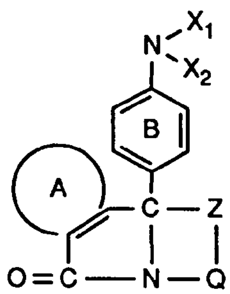

worin

der Ring A einen aromatischen oder heteroaromatischen Rest mit 6 Ringatomen, der einen aromatischen annellierten Ring aufweisen kann, wobei sowohl der Ring A als auch der annellierte Ring substituiert sein können;

der Ring B einen unsubstituierten oder durch Halogen, Niederalkyl, $C_1$-$C_{12}$-Alkoxy, besonders Niederalkoxy, Benzyloxy, Niederalkylcarbonylamino, Mono- oder Diniederalkylamino substituierten Benzolkern;

Z -NR, O oder S;

R Wasserstoff; unsubstituiertes oder durch Halogen, Hydroxy, Cyano, Benzoyl, $C_1$-$C_{12}$-Acyl, Diniederalkylamino oder Niederalkoxy substituiertes $C_1$-$C_{12}$-Alkyl; Cycloalkyl mit 5 bis 10 Kohlenstoffatomen; unsubstituiertes oder durch Halogen, Cyano, Nitro, $C_1$-$C_4$-Halogenalkyl, Niederalkyl, Niederalkoxy, Niederalkoxycarbonyl oder $C_1$-$C_{12}$-Acyl ringsubstituiertes Aryl oder Aralkyl; $C_1$-$C_{12}$-Acyl; N-Niederalkylcarbamoyl; oder gegebenenfalls ringsubstituiertes N-Arylcarbamoyl;

Q $C_1$-$C_{12}$-Alkylen, Aryl-$C_1$-$C_4$-Alkylen, 1,2-Cycloalkylen, 1,2- oder 1,8-Arylen oder Aralkylen; und

$X_1$ und $X_2$, unabhängig voneinander, je Wasserstoff; unsubstituiertes oder durch Halogen, Hydroxy, Cyano, Tetrahydrofuryl oder Niederalkoxy substituiertes Alkyl mit höchstens 12 Kohlenstoffatomen; Acyl mit 1 bis 8 Kohlenstoffatomen; Cycloalkyl mit 5 bis 10 Kohlenstoffatomen; oder unsubstituiertes oder durch Halogen, Cyano, Nitro, Trifluormethyl, Niederalkyl, Niederalkoxy, Niederalkoxycarbonyl, -NX′X″ oder 4-X′X″N-phenylamino ringsubstituiertes Aralkyl oder Aryl, worin X′ und X″, unabhängig voneinander, Wasserstoff, Niederalkyl, Cyclohexyl, Benzyl oder Phenyl darstellen; oder ($X_1$ und $X_2$) zusammen mit dem gemeinsamen Stickstoffatom einen fünf- oder sechsgliedrigen, vorzugsweise gesättigten, heterocyclischen Rest bedeuten.

2. Lactamverbindung gemäss Anspruch 1, dadurch gekennzeichnet, dass in Formel (1) der Ring A einen gegebenenfalls substituierten Benzol-, Naphthalin-, Pyridin-, Pyrazin-, Chinoxalin- oder Chinolinring darstellt.

3. Lactamverbindung gemäss Anspruch 1 oder 2, dadurch gekennzeichnet, dass in Formel (1)

Z — N-R

darstellt, worin R Wasserstoff, Niederalkyl, Cyano-Niederalkyl, Niederalkylcarbonyl, Phenyl, Benzyl, N-Niederalkylcarbamoyl oder gegebenenfalls durch Halogen, Nitro, Trifluormethyl, Niederalkyl, Niederalkoxy oder Niederalkoxycarbonyl substituiertes N-Phenylcarbamoyl bedeutet.

4. Lactamverbindung nach Anspruch 1, worin

A einen gegebenenfalls durch Halogen, Cyano, Nitro, $C_1$-$C_4$-Alkyl, $C_1$-$C_4$-Alkoxy, $C_1$-$C_4$-Alkylthio, $C_1$-$C_4$-Alkylcarbonyl, $C_1$-$C_4$-Alkoxycarbonyl, Amino, $C_1$-$C_4$-Alkylamino, Di-$C_1$-$C_4$-Alkylamino oder $C_1$-$C_4$-Alkylcarbonylamino substituierten Benzolring;

B einen unsubstituierten oder durch Halogen, $C_1$-$C_4$-Alkyl, $C_1$-$C_4$-Alkoxy, Benzyloxy, $C_1$-$C_4$-Alkylcarbonylamino, Mono- oder Di-$C_1$-$C_4$-Alkylamino substituierten Benzolkern;

Z -NR, O oder S;

R Wasserstoff; unsubstituiertes oder durch Halogen, Cyano, Benzoyl, $C_1$-$C_4$-Alkylcarbonyl, Phenoxysulfonyl, Phenylsulfonyl, $C_1$-$C_4$-Alkylsulfonyl, Di-$C_1$-$C_4$-Alkylamino oder $C_1$-$C_4$-Alkoxy substituiertes $C_1$-$C_4$-Alkyl; unsubstituiertes oder durch Halogen, Nitro oder Trifluormethyl, ringsubstituiertes Phenyl oder Benzyl; $C_1$-$C_4$-Alkyl-carbonyl; $C_1$-$C_4$-Alkyl-$SO_2$; $C_1$-$C_4$-Alkoxy-$SO_2$; N-$C_1$-$C_4$-alkylcarbamoyl; oder gegebenenfalls durch $C_1$-$C_4$-Alkyl, Halogen, $C_1$-$C_4$-Alkoxy, $C_1$-$C_4$-Halogenalkyl, Nitro, oder Cyano ringsubstituiertes N-Phenylcarbamoyl;

Q gegebenenfalls bis zu zweifach durch $C_1$-$C_4$-Alkyl, Phenyl, $C_5$-$C_6$-Spiroalkyl substituiertes $C_2$-$C_3$-Alkylen; 1,2-Cyclohexylen; 1,2-Cyclopentylen; 1,2-Phenylen; 1,8-Naphthylen; 1,2-Naphthylen; 1,3-Naphthylen; oder

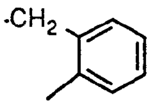 ;

und

$X_1$ und $X_2$, unabhängig voneinander, je Wasserstoff; unsubstituiertes oder durch $C_1$-$C_4$-Alkoxy, Hydroxy oder Cyano, substituiertes $C_1$-$C_4$-Alkyl; $C_1$-$C_4$-Alkylcarbonyl; oder ($X_1$ und $X_2$) zusammen mit dem gemeinsamen Stickstoffatom einen fünf- oder sechsgliedrigen gesättigten heterocyclischen Rest bedeuten.

5. Lactamverbindung gemäss Anspruch 1, der Formel

(2) 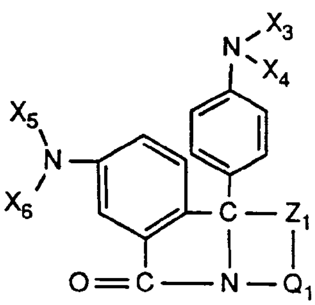

worin

$X_3$ und $X_4$ und $X_5$ und $X_6$ gleiche oder voneinander verschiedene Niederalkyl- oder Phenylreste darstellen; oder

$X_3$ und $X_4$ gemeinsam mit dem Stickstoffatom, an welches sie gebunden sind, Pyrrolidin;

$Q_1$ $C_2$-$C_3$-Alkylen, 1,2-Cyclohexylen, 1,2-Phenylen oder 1,8-Naphthylen;

$Z_1$ -O-, -S- oder -$NR_1$ ;

und

$R_1$ Wasserstoff, Phenyl, Benzyl, $C_1$-$C_4$-Alkyl, Cyano-$C_1$-$C_4$-Alkyl, $C_1$-$C_4$-Alkylcarbonyl, N-$C_1$-$C_4$-Alkylcarbamoyl oder gegebenenfalls durch Halogen, Nitro, Trifluormethyl, Niederalkyl oder Niederalkoxy substituiertes N-Phenylcarbamoyl bedeutet.

6. Lactamverbindung gemäss Anspruch 1, der Formel

(3)

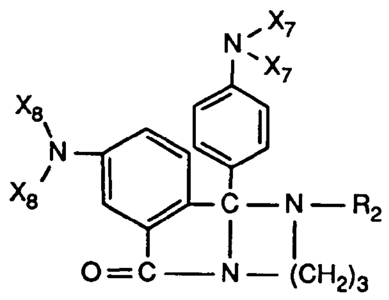

worin

$X_7$ und $X_8$, unabhängig voneinander, $C_1$-$C_4$-Alkyl und besonders Methyl und $R_2$ Wasserstoff, Phenyl, Niederalkyl oder Cyano-Niederalkyl bedeuten.

7. Verfahren zur Herstellung einer Lactamverbindung gemäss Anspruch 1, dadurch gekennzeichnet, dass man eine Ketosäure der Formel

(4)

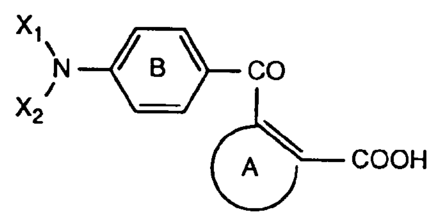

mit einer bifunktionellen Aminoverbindung der Formel

(5) $H_2N$-Q-Z-H

umsetzt, worin A, B, $X_1$, $X_2$, Q und Z die in Anspruch 1 angegebene Bedeutung haben.

8. Verwendung einer Lactamverbindung gemäss einem der Ansprüche 1 bis 15 als Farbbildnder in einem druckempfindlichen oder wärmeempfindlichen Aufzeichnungsmaterial.

9. Druck- oder wärmeempfindliches Aufzeichnungsmaterial, dadurch gekennzeichnet, dass es in seinem Farbreaktantensystem als Farbbildner mindestens eine Lactamverbindung gemäss einem der Ansprüche 1 bis 15 enthält.

10. Aufzeichnungsmaterial gemäss Anspruch 9, dadurch gekennzeichnet, dass die Lactamverbindung gemeinsam mit einem oder mehreren bekannten Farbbildnern enthalten ist.

**Patentansprüche für folgenden Vertragsstaat : ES**

1. Verfahren zur Herstellung einer Lactamverbindung der Formel

(1)

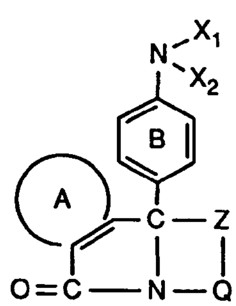

worin

der Ring A einen aromatischen oder heteroaromatischen Rest mit 6 Ringatomen, der einen aromatischen annellierten Ring aufweisen kann, wobei sowohl der Ring A als auch der annellierte Ring substituiert sein können;

der Ring B einen unsubstituierten oder durch Halogen, Niederalkyl, $C_1$-$C_{12}$-Alkoxy, besonders Niederalkoxy, Benzyloxy, Niederalkylcarbonylamino, Mono- oder Diniederalkylamino substituierten Benzolkern;

Z -NR, O oder S;

R Wasserstoff; unsubstituiertes oder durch Halogen, Hydroxy, Cyano, Benzoyl, $C_1$-$C_{12}$-Acyl, Diniederalkylamino oder Niederalkoxy substituiertes $C_1$-$C_{12}$-Alkyl; Cycloalkyl mit 5 bis 10 Kohlenstoffatomen; unsubstituiertes oder durch Halogen, Cyano, Nitro, $C_1$-$C_4$-Halogenalkyl, Niederalkyl, Niederalkoxy, Niederalkoxycarbonyl oder $C_1$-$C_{12}$-Acyl ringsubstituiertes Aryl oder Aralkyl; $C_1$-$C_{12}$-Acyl; N-Niederalkylcarbamoyl; oder gegebenenfalls ringsubstituiertes N-Arylcarbamoyl;

Q $C_1$-$C_{12}$-Alkylen, Aryl-$C_1$-$C_4$-Alkylen, 1,2-Cycloalkylen, 1,2- oder 1,8-Arylen oder Aralkylen; und

$X_1$ und $X_2$, unabhängig voneinander, je Wasserstoff; unsubstituiertes oder durch Halogen, Hydroxy, Cyano, Tetrahydrofuryl oder Niederalkoxy substituiertes Alkyl mit höchstens 12 Kohlenstoffatomen; Acyl mit 1 bis 8 Kohlenstoffatomen; Cycloalkyl mit 5 bis 10 Kohlenstoffatomen; oder unsubstituiertes oder durch Halogen, Cyano, Nitro, Trifluormethyl, Niederalkyl, Niederalkoxy, Niederalkoxycarbonyl, -NX′X″ oder 4-X′X″N-phenylamino ringsubstituiertes Aralkyl oder Aryl, worin X′ und X″, unabhängig voneinander, Wasserstoff, Niederalkyl, Cyclohexyl, Benzyl oder Phenyl darstellen; oder ($X_1$ und $X_2$) zusammen mit dem gemeinsamen Stickstoffatom einen fünf- oder sechsgliedrigen, vorzugsweise gesättigten, heterocyclischen Rest bedeutet,

dadurch gekennzeichnet, dass man eine Ketosäure der Formel

(4)

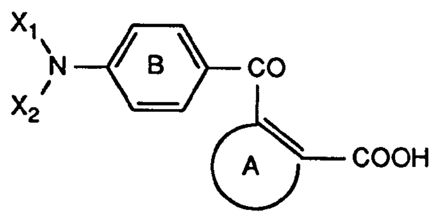

.

mit einer bifunktionellen Aminoverbindung der Formel

(5) $H_2N$-Q-Z-H

umsetzt, worin A, B, $X_1$, $X_2$, Q und Z wie zuvor definiert sind.

**2.** Verfahren gemäss Anspruch 1, zur Herstellung einer Lactamverbindung der Formel (1), worin der Ring A einen gegebenenfalls substituierten Benzol-, Naphthalin-, Pyridin-, Pyrazin-, Chinoxalin- oder Chinolinring darstellt.

**3.** Verfahren gemäss Anspruch 1 oder 2, zur Herstellung einer Lactamverbindung der Formel (1), worin

Z – N-R

und

R Wasserstoff, Niederalkyl, Cyano-Niederalkyl, Niederalkylcarbonyl, Phenyl, Benzyl, N-Niederalkylcarbamoyl oder gegebenenfalls durch Halogen, Nitro, Trifluormethyl, Niederalkyl, Niederalkoxy oder Niederalkoxycarbonyl substituiertes N-Phenylcarbamoyl bedeutet.

**4.** Verfahren gemäss Anspruch 1, zur Herstellung einer Lactamverbindung der Formel (1), worin

A einen gegebenenfalls durch Halogen, Cyano, Nitro, $C_1$-$C_4$-Alkyl, $C_1$-$C_4$-Alkoxy, $C_1$-$C_4$-Alkylthio, $C_1$-$C_4$-Alkylcarbonyl, $C_1$-$C_4$-Alkoxycarbonyl, Amino, $C_1$-$C_4$-Alkylamino, Di-$C_1$-$C_4$-Alkylamino oder $C_1$-$C_4$-Alkylcarbonylamino substituierten Benzolring;

B einen unsubstituierten oder durch Halogen, $C_1$-$C_4$-Alkyl, $C_1$-$C_4$-Alkoxy, Benzyloxy, $C_1$-$C_4$-Alkylcarbonylamino, Mono- oder Di-$C_1$-$C_4$-Alkylamino substituierten Benzolkern;

Z -NR, O oder S;

R Wasserstoff; unsubstituiertes oder durch Halogen, Cyano, Benzoyl, $C_1$-$C_4$-Alkylcarbonyl, Phenoxysulfonyl, Phenylsulfonyl, $C_1$-$C_4$-Alkylsulfonyl, Di-$C_1$-$C_4$-Alkylamino oder $C_1$-$C_4$-Alkoxy substituiertes $C_1$-$C_4$-Alkyl; unsubstituiertes oder durch Halogen, Nitro oder Trifluormethyl, ringsubstituiertes Phenyl oder Benzyl; $C_1$-$C_4$-Alkyl-carbonyl; $C_1$-$C_4$-Alkyl-$SO_2$; $C_1$-$C_4$-Alkoxy-$SO_2$; N-$C_1$-$C_4$-alkylcarbamoyl; oder gegebenenfalls durch $C_1$-$C_4$-Alkyl, Halogen, $C_1$-$C_4$-Alkoxy, $C_1$-$C_4$-Halogenalkyl, Nitro, oder Cyano ringsubstituiertes N-Phenylcarbamoyl;

Q gegebenenfalls bis zu zweifach durch $C_1$-$C_4$-Alkyl, Phenyl, $C_5$-$C_6$Spiroalkyl substituiertes $C_2$-$C_3$-Alkylen; 1,2-Cyclohexylen; 1,2-Cyclopentylen; 1,2-Phenylen; 1,8-Naphthylen; 1,2-Naphthylen; 1,3-Naphthylen; oder

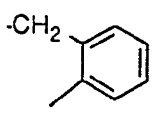 ;

und

$X_1$ und $X_2$, unabhängig voneinander, je Wasserstoff; unsubstituiertes oder durch $C_1$-$C_4$-Alkoxy, Hydroxy oder Cyano, substituiertes $C_1$-$C_4$-Alkyl; $C_1$-$C_4$-Alkylcarbonyl; oder ($X_1$ und $X_2$) zusammen mit dem gemeinsamen Stickstoffatom einen fünf- oder sechsgliedrigen gesättigten heterocyclischen Rest bedeuten.

**5.** Verfahren gemäss Anspruch 1, zur Herstellung einer Lactamverbindung der Formel (2),

(2) 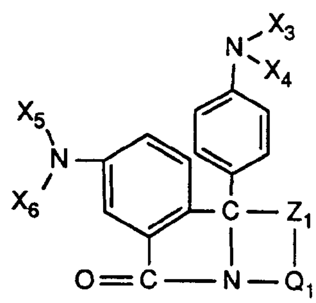 ,

worin

$X_3$ und $X_4$ und $X_5$ und $X_6$ gleiche oder voneinander verschiedene Niederalkyl- oder Phenylreste darstellen; oder

$X_3$ und $X_4$ gemeinsam mit dem Stickstoffatom, an welches sie gebunden sind, Pyrrolidin;

$Q_1$ $C_2$-$C_3$-Alkylen, 1,2-Cyclohexylen, 1,2-Phenylen oder 1,8-Naphthylen;

$Z_1$ -O-, -S- oder -$NR_1$ ;

und

$R_1$ Wasserstoff, Phenyl, Benzyl, $C_1$-$C_4$-Alkyl, Cyano-$C_1$-$C_4$-Alkyl, $C_1$-$C_4$-Alkylcarbonyl, N-$C_1$-$C_4$-Alkylcarbamoyl oder gegebenenfalls durch Halogen, Nitro, Trifluormethyl, Niederalkyl oder Niederalkoxy substituiertes N-Phenylcarbamoyl
bedeutet.

**6.** Verfahren gemäss Anspruch 1, zur Herstellung einer Lactamverbindung der Formel (3),

(3) 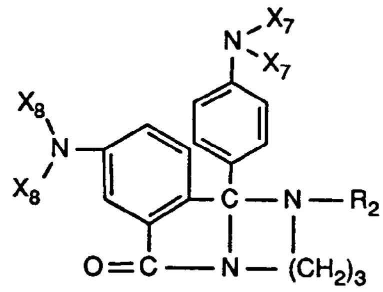

worin

$X_7$ und $X_8$, unabhängig voneinander, $C_1$-$C_4$-Alkyl und besonders Methyl und $R_2$ Wasserstoff, Phenyl, Niederalkyl oder Cyano-Niederalkyl bedeuten.

7. Verfahren zum Drucken oder Schreiben von Informationen auf einem druck- oder wärmempfindlichen Aufzeichungsmaterial, gekennzeichnet durch die Verwendung eines Farbbildners der Formel (1), worin die Substituenten A, B, $X_1$, $X_2$, Q und Z wie in einem der Ansprüche 1 bis 6 definiert sind.

8. Verfahren zur Herstellung eines druck- oder wärmeempfindliches Aufzeichnungsmaterials, dadurch gekennzeichnet, dass man in das Farbreaktantensystem als Farbbildner mindestens eine Lactamverbindung der Formel (1), worin die Substituenten A, B, $X_1$, $X_2$, Q und Z wie in einem der Ansprüche 1 bis 6 definiert sind, einarbeitet.

9. Verfahren gemäss Anspruch 8, dadurch gekennzeichnet, dass man neben einer Lactamverbindung der Formel (1) noch einen oder mehrere bekannte Farbbildnern einarbeitet.

Europäisches Patentamt

# EUROPÄISCHER RECHERCHENBERICHT

Nummer der Anmeldung

EP 91810713.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| X | US - A - 3 336 306 (T.S. SULKOVSKI) * Ansprüche 1,5,11; Spalte 1, Zeilen 9-49,57-72 * -- | 1,2,4, 7 | C 07 D 487/04<br>C 07 D 498/04<br>C 07 D 513/04<br>B 41 M 5/26<br>B 41 M 5/28<br>B 41 M 5/34 |
| X | US - A - 3 470 180 (W.J. HOULIHAN) * Anspruch 2; Spalte 1, Zeilen 29-63; Spalte 2, Zeilen 49-66 * -- | 1-4,7 | |
| X | DD - A - 51 854 (J.R. GEIGY AG) * Anspruch 1; Beispiel 1 * -- | 1,2,4, 5,7 | |
| A | US - A - 3 591 599 (H. HOEHN et al.) * Spalte 1, Zeile 1 - Spalte 2, Zeile 13 * -- | 1,2,4, 5,7 | |
| A | US - A - 3 509 147 (W.J. HOULIHAN) * Anspruch 1 * -- | 1 | RECHERCHIERTE SACHGEBIETE (Int. Cl 5) |
| A | DE - A - 1 670 446 (CHEMISCHE FABRIK VON HEYDEN GMBH) * Ansprüche 1,2 * -- | 1,2,4, 5,7 | C 07 D 487/00<br>C 07 D 498/00<br>C 07 D 513/00 |
| A | DE - A - 3 008 494 (APPLETON PAPERS INC.) * Ansprüche 1,3-5,135-137 * ---- | 1,8,9 | |

Der vorliegende Recherchenbericht wurde für alle Patentansprüche erstellt.

| Recherchenort | Abschlußdatum der Recherche | Prüfer |
|---|---|---|
| WIEN | 20-12-1991 | MAZZUCCO |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82